# EUROPEAN PATENT APPLICATION

(11) **EP 3 673 793 A1**
(43) Date of publication of application: **01.07.2020**
(21) Application number: 19200227.7
(22) Date of filing: 27.09.2019
(51) Int. Cl.: A61B 5/00, A61B 5/06

(54) **GENERATION OF AN ULTRA-HIGH DEFINITION MAP OF ELECTROMAGNETIC FIELDS**

(30) Priority: 28.09.2018 US 201862738148 P; 18.01.2019 US 201962794209 P; 19.09.2019 US 201916576568
(71) Applicant: Covidien LP, Mansfield, MA 02048 (US)
(72) Inventor: KOYRAKH, Lev A., Plymouth, MN Minnesota 55442 (US); BARAK, Ron, 6920024 Tel Aviv (IL); BIRENBAUM, Ariel, 4672559 Herzliya (IL); WEINGARTEN, Oren P., 4672559 Herzliya (IL)
(74) Representative: Maschio, Antonio

(57) **Abstract**

A method of determining a position of an electromagnetic (EM) sensor. The method including generating a magnetic field, storing in memory an ultra-high definition (UHD) map of the magnetic field, and placing an EM sensor within the magnetic field. The method further includes sampling the magnetic field with the EM sensor at one point, conducting a global search of the UHD map of the magnetic field to identify a point in the magnetic field with the smallest difference from the point sampled by the EM sensor, conducting a local search of a portion of the UHD map in proximity to identified point to determine a new point in the magnetic field with the smallest difference from the point sampled by the EM sensor, and iterating the local search until the difference between the new point in the magnetic field and the sampled point is below a threshold.

## Description

### CROSS REFERENCE TO RELATED APPLICATION

The present application claims the benefit of and priority to U.S. Provisional Application Serial No. 62/794,209, filed on January 18, 2019, and 62/738,148, filed on September 28, 2018 the entire contents of which are incorporated herein by reference.

### Technical Field

This disclosure relates generally to electromagnetic navigation systems and methods. More particularly, this disclosure is directed to systems and methods for detecting the location of a single sensor within an electromagnetic field.

### Background

Electromagnetic navigation (EMN) has helped expand medical imaging, diagnosis, prognosis, and treatment capabilities by enabling a location and/or an orientation of a medical device and/or of a target of interest to be accurately determined within a patient's body. Generally, an antenna generates an electromagnetic (EM) field in an EM volume, a sensor incorporated onto a medical device senses an EM signal or strength based on the field, and the EMN system identifies a sensor location based on the sensed EM strength. The EM strength at each location in the EM volume is previously measured or mapped to enable the sensor location to be identified in the EM volume by comparing the sensed EM strength and the previously measured EM strength.

Given the foregoing, there is a need for improvements to EMN systems and methods for generating a map for electromagnetic navigation and localization of sensors therein.

### Summary

The disclosure is related to systems and methods for generating an ultra-high definition (UHD) map of EM fields. In one aspect the method includes calculating a simulated UHD map based on the geometry and architecture of a plurality of magnetic field antennae, generating a magnetic field with the plurality of magnetic field antennae, and detecting the magnetic fields generated by the plurality of magnetic field antennae at a plurality of positions within the generated magnetic field. The method further includes calculating a difference between an expected magnetic field and the detected magnetic field at each of the plurality of positions to identify any interference at the plurality of positions, interpolating the calculated difference between each of the plurality of positions to generate a UHD interference map and adding the UHD interference map to the simulated UHD map.

The method may be performed in a clinical suite after placing the plurality of magnetic field antennae on a surgical bed. The surgical bed may include metal components, and the generation of magnetic fields in proximity of the metal components may induce eddy currents in the metal components which contribute to the detected interference.

In accordance with the method adding the UHD interference map to the simulated UHD map generates an in-situ UHD map. The in-situ UHD map may have a resolution of about 1 mm and may be stored in a memory accessible by one or more components of an electromagnetic navigation system. The in-situ UHD map may be used to determine the position of a sensor placed within the magnetic field generated by the plurality of magnetic field antennae.

A further aspect of the disclosure is a method of determining a position of an electromagnetic (EM) sensor. The method includes generating a magnetic field with a plurality of magnetic field antennae, storing in memory an ultra-high definition (UHD) map of the magnetic field generated by the plurality of magnetic field antennae, and placing an EM sensor within the magnetic field. The method further includes sampling the plurality of magnetic fields with the EM sensor at one point, conducting a global search of the UHD map of the magnetic field to identify a point in the magnetic field with the smallest difference from the point sampled by the EM sensor, and conducting a local search of a portion of the UHD map in proximity to identified point to determine a new point in the magnetic field with the smallest difference from the point sampled by the EM sensor. The method further includes iterating the local search until the difference between the new point in the magnetic field and the sampled point is below a threshold.

The method may revert to a global search upon determination that there is no convergence during the iteration. Additionally, at predetermined times a global search may be performed.

In accordance with a further aspect upon determination that difference between the new point in the magnetic field and the sampled point is below a threshold, the new point is stored as the location and orientation of the EM sensor within the magnetic field. The stored location and orientation of the EM sensor may be used as the starting point for a further local search to identify the next location of the EM sensor.

In accordance with another aspect, the location and orientation of the EM sensor is output to a user-interface for display to a user. The location and orientation of the EM sensor may be displayed in a 3D model that has been registered to the magnetic field. The 3D model may be of at least a portion of a patient's anatomy, and the EM sensor is located within the portion of the patient's anatomy from which the 3D model was made.

In accordance with another aspect, the UHD map is an in-situ UHD map that accounts for interference caused by placement of the plurality of magnetic field antennae in proximity of a metal object.

Still further, in accordance with another aspect, the global search may be performed using a subset of the UHD map which approximates a lower resolution map.

### Brief Description of the Drawings

Objects and features of the presently disclosed systems and methods will become apparent to those of ordinary skill in the art when descriptions of various embodiments are read with reference to the accompanying drawings, of which:
FIG. 1 shows an example electromagnetic navigation (EMN) system, in accordance with the disclosure;
FIG. 2 is a perspective view of a magnetic field generator having multiple antennae, in accordance with the disclosure;
FIG. 3 is a flow chart of a method of forming an in-situ ultra-high definition map of the magnetic field generated by the magnetic field generator of FIG. 2; and

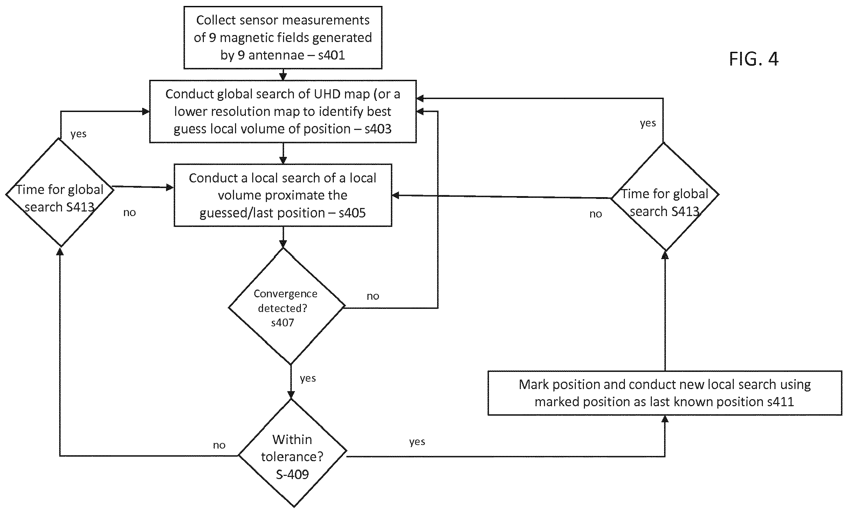

FIG. 4 is a flow chart of a method of determining a position of an electromagnetic sensor placed in a magnetic field generated by the magnetic field generator of FIG. 2.

### DETAILED DESCRIPTION

The present disclosure is related to systems and methods for generating an ultra-high density (HD) map and identifying a location and/or an orientation of a sensor, which may include at least one coil, based on the UHD map. In some aspects, the respective geometric configurations the antennas enable automated and highly repeatable processes for reproducing such antennas and/or for mathematically calculating the expected or theoretical EM strength at every HD grid point within an EM volume (for instance, where the antennas have geometric configurations based on linear portions of printed circuit board (PCB) traces, which facilitate use of the superposition principle in computing the total contribution of the fields generated by way of each antenna to the total combined EM field within the volume). These mathematical calculations may be combined with actual measurements made in a coarse coordinate system, which includes fewer grid points than the number of grid points used for the mathematically calculated EM strength.

Further, the present disclosure is related to systems and methods for identifying a location and/or an orientation of an EM sensor using the UHD map. In general, the EM sensor senses EM strengths, and an EMN system compares the sensed EM strengths with the expected EM strengths of the UHD map and identifies the location and the orientation of the EM sensor.

In an aspect of the present disclosure a combined global and local search algorithm is employed to iteratively define the most likely positions of the EM sensor in the magnetic field by comparing the detected signals to locations in the UHD map.

In some cases, it may be desirable for the sensor to be a small-sized sensor, such as a single-coil sensor, because, for instance, a small sized sensor may be navigable to additional locations (e.g., narrower portions of a luminal network) within the patient, to which a larger-sized sensor may not be navigable. Additionally, in contrast to large-size sensors which sometimes must be removed from the patient during a procedure to make room in a working channel for other tools, the small-sized sensor may be incorporated into a catheter or other tool and thus remain within the patient throughout the procedure without interfering with the other tools, thereby facilitating EMN functionality throughout the procedure.

To enable a small-sized sensor such as a single-coil sensor to be accurately located within an EM volume, it may be necessary to generate multiple (for instance, 6, or 9 or more) geometrically diverse EM fields within the EM volume. However, because each of the EM fields would require generation of a measured mapping of the corresponding EM strength at each location in the EM volume, increasing the number of EM fields increases the number of mappings, which can be time consuming and laborious. Additionally, to improve the accuracy with which the sensor location can be determined, precise measurements at many (for example, thousands) of grid points within the EM volume are needed, which could make the generating of the mapping even more time consuming. Also, because of the potential variability during the manufacturing processes and tolerances of electrical equipment, the mapping process may need to be completed for each new antenna that is produced and for each electromagnetic navigation system installation.

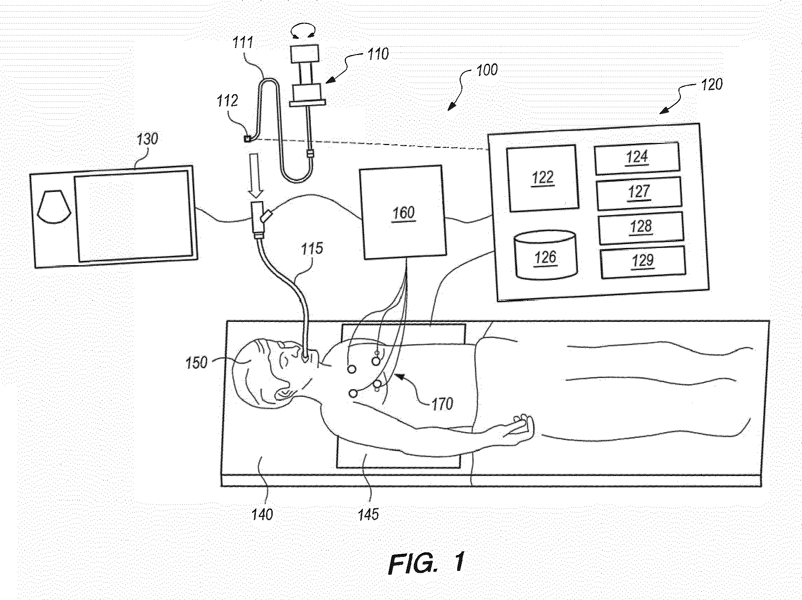

FIG. 1 illustrates an example electromagnetic navigation (EMN) system 100, which is configured to identify a location and/or an orientation of a medical device, or sensor thereof, navigating (e.g., to a target) within the patient's body by using an antenna assembly, which includes a plurality of antennas and generates EM fields. The EMN system 100 is further configured to augment CT, MRI, or fluoroscopic images in navigation through patient's body toward a target of interest, such as a deceased portion in a luminal network of a patient's lung.

The EMN system 100 includes a catheter guide assembly 110, a bronchoscope 115, a computing device 120, a monitoring device 130, an EM board 140, a tracking device 160, and reference sensors 170. The bronchoscope 115 is operatively coupled to the computing device 120 and the monitoring device 130 via a wired connection (as shown in FIG. 1) or wireless connection (not shown).

The bronchoscope 115 is inserted into the mouth of a patient 150 and captures images of the luminal network of the lung. In the EMN system 100, inserted into the bronchoscope 115 is a catheter guide assembly 110 for achieving access to the periphery of the luminal network of the lung of the patient 150. The catheter guide assembly 110 may include an extended working channel (EWC) 111 with an EM sensor 112 at the distal portion of the EWC 111. A locatable guide catheter (LG) may be inserted into the EWC 111 with another EM sensor at the distal portion of the LG. The EM sensor 112 at the distal portion of the EWC 111 or the LG is used to identify a location and/or an orientation of the EWC 111 or the LG while navigating through the luminal network of the lung. Due to the size restriction in the EWC 111 or the LG, in some embodiments, the EM sensor 112 may include only one single coil for detecting EM strength of an EM field over the patient 150. However, the number of coils in the EM sensor is not limited to one but may be two or more.

The computing device 120, such as, a laptop, desktop, tablet, or other similar computing device, includes a display 122, one or more processors 124, memory 126, an AC current driver 127 for providing AC current signals to the antenna assembly 145, a network card 128, and an input device 129. The particular configuration of the computing device 120 illustrated in FIG. 1 is provided as an example, but other configurations of the components shown in FIG. 1 as being included in the computing device 120 are also contemplated. In particular, in some embodiments, one or more of the components (122, 124, 126, 127, 128, and/or 129) shown in FIG. 1 as being included in the computing device 120 may instead be separate from the computing device 120 and may be coupled to the computing device 120 and/or to any other component(s) of the system 100 by way of one or more respective wired or wireless path(s) to facilitate the transmission of power and/or data signals throughout the system 100. For example, although not shown in FIG. 1, the AC current driver 127 may, in some example aspects, be separate from the computing device 120 and may be coupled to the antenna assembly 145 and/or coupled to one or more components of the computing device 120, such as the processor 124 and the memory 126, by way of one or more corresponding paths.

The EMN system 100 may also include multiple computing devices, wherein the multiple computing devices are employed for planning, treatment, visualization, or helping clinicians in a manner suitable for medical operations. The display 122 may be touch-sensitive and/or voice-activated, enabling the display 122 to serve as both input and output devices. The display 122 may display two-dimensional (2D) images or three-dimensional (3D) model of a lung to locate and identify a portion of the lung that displays symptoms of lung diseases.

The one or more processors 124 execute computer-executable instructions. The processors 124 may perform image-processing functions so that the 3D model of the lung can be displayed on the display 122 or location algorithm to identify a location and an orientation of the EM sensor 112. In embodiments, the computing device 120 may further include a separate graphic accelerator (not shown) that performs only the image-processing functions so that the one or more processors 124 may be available for other programs. The memory 126 stores data and programs. For example, data may be mapping data for the EMN or any other related data such as a HD map, image data, patients' medical records, prescriptions and/or history of the patient's diseases.

The HD map may include a plurality of grid points in a fine coordinate system of the EM volume in which a medical device (e.g., the EWC 111, LG, treatment probe, or other surgical devices) is to be navigated, and expected EM strengths at each of the plurality of grid points. When the EM sensor 112 senses EM strength at a point, the one or more processors 124 may compare the sensed EM strength with the expected EM strengths in the HD map and identify the location of the EM sensor 112 within the EM volume. Further, an orientation of the medical device may be also calculated based on the sensed EM strength and the expected EM strengths in the HD map.

As shown in FIG. 1, the EM board 140 is configured to provide a flat surface for the patient 150 to lie upon and includes an antenna assembly 145. When the patient 150 lies upon on the EM board 140, the antenna assembly 145 generates an EM field sufficient to surround a portion of the patient 150 or the EM volume. The antenna assembly 145 includes a plurality of antennas, each of which may include a plurality of loops. In one aspect, each antenna is configured to generate an EM waveform having a corresponding frequency. The number of antennas may be at least six. In an aspect, the number of antennas may be nine so that nine different EM waveforms can be generated.

In another aspect, a time multiplexing method is employed in generating the EM waveforms. For example, the antennas of the antenna assembly 145 may generate EM waveforms with the same frequency at different times during a period. In another aspect, frequency multiplexing method may be employed, where each antenna generates EM waveform having a frequency different from each other. In still another aspect, combination of the time multiplexing and frequency multiplexing methods may be employed. The antennas are grouped into more than one group. Antennas in the same group generate EM waveforms having the same frequency but at different times. Antennas in different groups may generate EM waveforms having different frequencies from each other. Corresponding de-multiplexing method is to be used to separate EM waveforms.

In an aspect, each antenna may have a geometric configuration (for instance, where the antennas each have geometric configurations based on linear portions of printed circuit board (PCB) traces or wires, which facilitate use of the superposition principle in computing the total contribution of the fields generated by way of each antenna to the total combined EM field within the volume) so that each portion of the plurality of loops can be expressed as mathematical relationship or equations, as described in further detail below. The magnetic field can thus be computed for each trace on the antenna and the contributions from all traces can be summed. Based on this geometric configuration, expected EM strength at each grid point in the HD map can be theoretically or mathematically calculated.

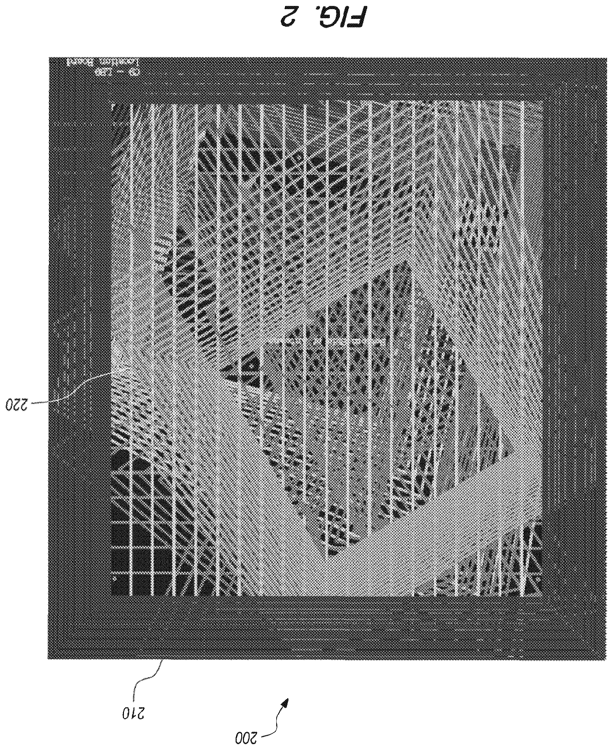

An example of the design of the antenna assembly is made with reference to FIG. 2, which is a graphical illustration of an example antenna assembly layout 200 of the antenna assembly 145 of the EMN system 100, according to an embodiment of the present disclosure. The antenna assembly layout 200 includes a substrate 210, such as a printed circuit board (PCB), which is formed of an electrically insulating material and may include one or more layers. The antenna assembly layout 200 also includes multiple planar antennas 220, which are formed of an electrically conductive material, such as a PCB trace, deposited on the substrate 210 and arranged in multiple loops or as a coil. In one example, each of the planar antennas 220 is deposited on a respective one of the layers of the substrate 210. In the example antenna assembly layout 200 of FIG. 2, multiple layers of the substrate 210 are shown simultaneously.

Each of the multiple antennas may be configured to radiate a separate EM field, for example, using frequency division multiplexing and/or time division multiplexing controlled by the processors 124 or by another generator. For example, the antennas, in some aspects, may be configured to radiate multiple EM fields sufficient in number and/or sufficient in diversity of characteristics (such as frequency, time, modulation scheme, and/or the like) to enable a single-coil electromagnetic sensor mounted on the EWC 111, or on any other medical device, to be used to determine the location and/or the orientation of the sensor, the EWC 111, and/or the medical device. The antenna assembly 145 may, as noted above, include six to nine or more loop antennas. In some embodiments, for each of the loop antennas, the distances between its adjacent loops increase as the loops become larger. For example, for each of the planar antennas, respective distances between adjacent pairs of loops may increase in a direction from an innermost one of the loops to an outermost one of the loops of the respective planar antenna. In various embodiments, two or more of the loop antennas of the antenna assembly 145 may have a same number of loops or may have respectively different numbers of loops. Additional aspects of such example antennas and methods of manufacturing the antennas are disclosed in U.S. Patent Application No. 15/337,056, entitled "ELECTROMAGNETIC NAVIGATION ANTENNA ASSEMBLY AND ELECTROMAGNETIC NAVIGATION SYSTEM INCLUDING THE SAME," filed on October 28, 2016 and published as U.S. 2018/0123248, the entire contents of which are hereby incorporated by reference herein.

The memory 126 may store data and programs related to identification of a location and an orientation. The data includes a high density (HD) map, which includes a plurality of grid points according to the fine coordinate system for the EM volume and expected EM strengths at the grid points. The HD map may be based on three-axis coordinate system, where each grid point has three coordinates corresponding to the three axes, respectively. In this case, the expected EM strength at each grid point may include one EM strength value along each axis for each EM waveform. For example, if there are nine antennas generating nine different EM waveforms, each of which having a separate frequency, and three axes are x, y, and z axes, the expected EM strength may include nine EM strength values along the x axis, nine EM strength values along the y axis, and nine EM strength values along the z axis, at each grid point. Such expected EM strength at each grid point may be expressed in a nine by three matrix form.

The HD map may be made with computations, which includes theoretically calculated EM strengths at each axis at each grid point in the fine coordinate system, and measurement, which includes measurements at each axis at each grid point in the coarse coordinate system. The fine coordinate system includes all the grid points in the coarse coordinate system and the grid points of the fine coordinate system are more finely distributed than those of the coarse coordinate system. By using the geometric configuration of the antennas of the antenna assembly 145, measurement may not have to be made with the fine coordinate system. Rather, the measurement may be made in the coarse coordinate system and theoretical computations may be made in the fine coordinate system. By combining the measurements in the coarse coordinate system with the theoretical computations in the fine coordinate system, the HD map may be generated.

After passage of time or due to foreign objects near the EMN system 100, measurements by the EM sensor 112 or other hardware may need to be calibrated. Such calibration data may be also stored in the memory 126 in a form of sensor calibration and hardware calibration.

The location search algorithm may utilize any error minimization algorithm in identifying the location and the orientation of the EM sensor 112. For example, the Levenberg-Marquardt algorithm may be employed to minimize errors between the EM strengths of the HD map and the sensed EM strengths. Other error minimization methods or algorithms, which a person having ordinary skill in the art can readily appreciate, may also be utilized without departing from the scope of this disclosure. Further details of the HD map generation and error correction can be found in commonly assigned U.S. Application No. 15/5337,166 filed October 28, 2016 and entitled SYSTEM AND METHOD FOR IDENTIFYING A LOCATION AND/OR AN ORIENTATION OF AN ELECTROMAGNETIC SENSOR BASED ON A MAP and published as U.S. 2018/0116730, the entire contents of which are incorporated herein by reference.

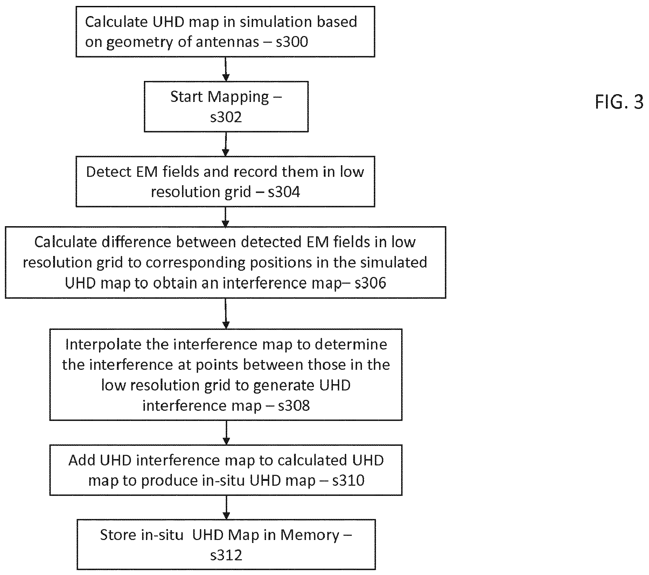

The HD map, as noted above, provides a grid of positions (e.g., every 5 mm). Each grid position includes as many vectors as there are antennas in the antenna assembly 145. Thus, if there are nine antennae, there will be 9 magnetic field vectors at each grid position. In accordance with the disclosure, a method for defining an ultra-HD (UHD) map, which provides a high-resolution grid of positions (e.g., every 1 mm), for a given system is described. The starting point for the UHD map is a generating a calculated or simulated UHD map (step 300 in Fig. 3) that can be derived from the construction or geometry of the antenna assembly 145 and the driving frequencies of the individual antenna using the Biot-Savart law for mapping magnetic fields, as described above.

In the process of installing an individual EM board 140 (including antenna assembly 145) as part of the EMN system 100 in a specific location, an in-situ mapping of the EM field must be undertaken. This in-situ mapping is necessary to account for eddy currents that can be induced in the metal frames of surgical beds and other metal components that are within the magnetic field. These eddy currents in the metal components, particularly the surgical bed, generate their own magnetic fields, referred to herein as additive magnetic fields. These additive magnetic fields are at the same frequencies as the magnetic fields generated by the antenna assembly 145.

In accordance with the generation of the UHD map, mapping is started at step 302 and actual readings of the EM fields generated by the nine antennae are taken using an EM field sensor are detected at step 304. These readings are taken specific positions forming a low-resolution grid of positions. In one example, the low-resolution grid is a 9 × 9 × 9 matrix of positions. The EM field measurements at each of these positions is compared by the processor 124 to that of the simulated UHD map. The difference between the detected magnetic fields of the low-resolution gird and the simulated magnetic field expected in the simulated UHD map at each of the positions of the low-resolution grid, represents the interference (e.g., bed interference) that is generated by the eddy currents (the additive magnetic fields) and is calculated at step 306.

The low-resolution grid (i.e., the sampling points) is spaced over the entirety of the EM board 140. Because the interference or additive magnetic fields do not dramatically change between the sample points, the processor 124 is able to interpolate, at step 308, the low-resolution bed interference map into a high-resolution interference map having the same number of positions as the calculated UHD map to generate a UHD interference map. The UHD map of the interference is then added to the simulated UHD map to provide an in-situ UHD map at step 310. This in-situ UHD map is saved in memory at step 312 and is then used for future navigation using the EMN system 100. Because the UHD interference map is added to the calculated UHD map, accurate positions of an EM sensor placed in the in-situ UHD map can be obtained. Effectively, this removes the undesirable effect of the detected interference caused by the metal in the surgical bed or from other sources that are largely static in the clinical space.

In at least one embodiment the sensor that is used to sample the low-density matrix is a three-coil sensor, though other sensors may be used without departing from the scope of the present disclosure.

In further embodiments, a magnetic field transform is determined. The transform may be a diagonal matrix or a near diagonal matrix. A diagonal matrix allows freedom for cross-talking between the coils of the sensor. In addition, the computation allows each antenna in the location board 140 a slight independent movement. Multiplication of the magnetic field of the simulated HD map by the transform should result in a similar UHD map as determined by the process described above.

Now that the UHD map has been generated, the UHD map can be used for navigation of the catheter guide assembly 110, and specifically the EM sensor 112 at the distal portion of the EWC 111 or in the LG to a desired location within lungs of the patient. The EM sensor 112 is used to detect the EM field. The output of the sensor 112 is compared to the UHD map to determine a position within the EM field. This position in the EM field may then be used to determine a position within a 3D model that has been registered with the patient and the EM field so that a position within the patient can be graphically depicted.

As noted above, the catheter guide assembly may have an EM sensor 112 located on the EWC 111. In one embodiment this is a single coil 5 degrees of freedom (DOF) sensor. IN contrast, the EM sensor 112 in the LG typically includes six coils which allow for resolution of 6 DOF.

During navigation, and indeed anytime the EM sensor 112 is within the EM field generated by the EM board 140, the processor 14 receives signals generated by the EM sensor 112 and the position of the EM sensor in the magnetic field is determined and stored in memory. During normal operation the EM sensor 112 is constantly outputting detected magnetic field information to the processor 124, however, it is the detected magnetic field information from the LG that is used for navigation. Once proximate a target or region of interest it is common for the LG to be removed from the EWC 111 leaving just the EM sensor 112 on the EWC 111 to provide position information.

Upon removal of the LG from the catheter guide assembly 110, which may be determined by a rapid change in position output by LG, the processor 124 can determine that only the sensor 112 in the EWC should be used for further navigation and position determination.

Position determination using the EM sensor 112 in the EWC 111 can be performed using a search algorithm such as the Levenberg-Marquadt search algorithm. The search algorithm searches for a position in the UHD map at which the difference between the magnetic field and the magnetic field detected by the EM sensor 112 is minimized (e.g., within some tolerance). Specifically, the search algorithm iterates starting from a known or best guessed position to find a position and direction of the EM sensor 112 coil which minimizes the difference between the 9 measured magnetic fields and the projection of 9 magnetic fields in the found position on the found direction of the UHD map.

This process of searching can be broken into two parts: the first is a global search and the second is a local search. The global search seeks to identify a portion of the EM field with the most likely position solution. The global search can also provide a best guess position of the EM sensor 112. The local search is very similar in terms of the search. The primary difference is the volume of the EM field that is being searched.

Whether a global or a local search, the search starts with a best guess of position. The best guess of position can be either the last found position or a position on the UHD map, whichever has smaller differences to the measured signals. For a global search, rather than using the in-situ UHD map described above, a lower resolution map of the EM field may be employed, for example one consistent with the HD map that only had grid points every 5 mm in the EM field. In this way a general concept of the proximate location of the position can be quickly identified and the process can move to the local search. This global search of these positions is typically searched in parallel using a Graphic Processor Unit (GPU). This may be run in the background of the application running on the processors 124.

The local search is similar except the starting point is always the last found position. Again, the Levenberg-Marquadt algorithm is employed to conduct a search. The nine magnetic fields at that position (generated by the EM board 140) are sampled, and the sampled signals of the magnetic fields are interpolated on the UHD map. Next a set of equations is employed to solve for the sensor direction that when projecting the nine magnetic fields on that direction in the checked position identifies the grid points in the UHD map that are closest to the measured signals. This process is then iterated until the differences between the measured magnetic fields and the magnetic fields associated with positions on the UHD map are minimized within some tolerance. The result is the position of the EM sensor 112 within the EM field, which as noted above, can be converted into a position within a 3D model that has been registered to the patient and EM field. As the EM sensor 112 moves within the EM field, this process is repeated on a constant loop such that the location of the EM sensor 112 is continually tracked.

The process can be of conducting the global and local search can be described as follows. At step 401 collect signals from the nine antennae using the EM sensor 112 on the EWC 111. Next at step 403, conduct a global search of the UHD map to identify a best guess of the location of the EM sensor 112 within the EM field. That is this is a position that is within some tolerance of likelihood. As noted above, this may be performed using the Levenberg-Marquadt search algorithm. This yields a subset of the UHD map (i.e., a local volume) within which the search will be iterated. Within this local volume of the UHD map the local search will be conducted at step 405. The local search of the local volume is iterated until a match is made between the detected signals and a position on the UHD map that is within some tolerance at step 409. Once within the tolerance the position of the EM sensor 112 within the UHD map is determined. As the local search is iterated, if at step 407 it is determined that there is no convergence between the detected signals and the resolution of the local search, the local search may be ended, and a new global search conducted.

Once the position is determined at step 409, this position is used as the last known position for conducting a renewed local search at step 411 and the process continues to iterate and update the detected position of the EM sensor 112. In at least one embodiment, the global search may be periodically conducted as depicted in step 413.

By using the process described herein accurate navigation of the EWC 111 may be continued even after the removal of the LG and its sensors. The EM sensor 112 on the EWC may only be a 5 DOF sensor, but typically at the point in the procedure where it would be employed the amount of movement of the EWC to reach a desired target is relatively small, measured in a few cm. The primary purpose of the EM sensor in the EWC is to give continued update of the position of the EWC 111 while the LG is removed and tools such as a biopsy tool is inserted this helps ensure that the tools will reach the desired target and if the EWC 111 through which the tools move has been displaced, the clinician can be alerted.

Although embodiments have been described in detail with reference to the accompanying drawings for the purpose of illustration and description, it is to be understood that the inventive processes and apparatus are not to be construed as limited. It will be apparent to those of ordinary skill in the art that various modifications to the foregoing embodiments may be made without departing from the scope of the disclosure. For example, various steps of the methods described herein may be implemented concurrently and/or in an order different from the example order(s) described herein.

The invention may be described by reference to the following numbered paragraphs:-
1. A method of generating an ultra-high definition (HUD) map of EM fields comprising:
   calculating a simulated UHD map based on the geometry and architecture of a plurality of magnetic field antennae;
   generating a magnetic field with the plurality of magnetic field antennae;
   detecting the magnetic fields generated by the plurality of magnetic field antennae at a plurality of positions within the generated magnetic field;
   calculate a difference between an expected magnetic field and the detected magnetic field at each of the plurality of positions to identify any interference at the plurality of positions;
   interpolating the calculated difference between each of the plurality of positions to generate a UHD interference map; and
   adding the UHD interference map to the simulated UHD map.
2. The method of paragraph 1, wherein the method is performed in a clinical suite;
3. The method of paragraph 1, further comprising placing the plurality of magnetic field antennae on a surgical bed.
4. The method of paragraph 3, wherein the surgical bed incudes metal components.
5. The method of paragraph 4, wherein the generation of magnetic fields in proximity of the metal components induces eddy currents in the metal components which contribute to the detected interference.
6. The method of paragraph 1, wherein adding the UHD interference map to the simulated UHD map generates an in-situ UHD map.
7. The method of paragraph 6, wherein the in-situ UHD map has a resolution of about 1 mm.
8. The method of paragraph 6, wherein the in-situ UHD map is stored in a memory accessible by one or more components of an electromagnetic navigation system.
9. The method of paragraph 8, wherein the in-situ UHD map is used to determine the position of a sensor placed within the magnetic field generated by the plurality of magnetic field antennae.
10. A method of determining a position of an electromagnetic (EM) sensor comprising:
   generating a magnetic field with a plurality of magnetic field antennae;
   storing in memory an ultra-high definition (UHD) map of the magnetic field generated by the plurality of magnetic field antennae;
   placing an EM sensor within the magnetic field;
   sampling the magnetic field with the EM sensor at one point;
   conducting a global search of the UHD map of the magnetic field to identify a point in the magnetic field with the smallest difference from the point sampled by the EM sensor;
   conducting a local search of a portion of the UHD map in proximity to identified point to determine a new point in the magnetic field with the smallest difference from the point sampled by the EM sensor;
   iterating the local search until the difference between the new point in the magnetic field and the sampled point is below a threshold.
11. The method of paragraph 10, wherein upon determining that there is no convergence during the iteration of the local search, a new global search is conducted.
12. The method of paragraph 10, wherein at predetermined times a global search is performed.
13. The method of paragraph 10, wherein upon determination that difference between the new point in the magnetic field and the sampled point is below a threshold, the new point is stored as the location and orientation of the EM sensor within the magnetic field.
14. The method of paragraph 13, wherein the stored location and orientation of the EM sensor is used as the starting point for a further local search to identify the next location and orientation of the EM sensor.
15. The method of paragraph 14, wherein the location and orientation of the EM sensor is output to a user-interface for display to a user.
16. The method of paragraph 15, wherein the location and orientation of the EM sensor is displayed in a 3D model that has been registered to the magnetic field.
17. The method of paragraph 16, wherein the 3D model is of at least a portion of a patient's anatomy.
18. The method of paragraph 17, wherein the EM sensor is located within the portion of the patient's anatomy from which the 3D model was made.
19. The method of paragraph 10, wherein the UHD map is an in-situ UHD map that accounts for interference caused by placement of the plurality of magnetic field antennae in proximity of a metal object.
20. The method of paragraph 10, wherein the global search is performed using a subset of the UHD map which approximates a lower resolution map.

## Claims

1. A method of generating an ultra-high definition (HUD) map of EM fields comprising:
calculating a simulated UHD map based on the geometry and architecture of a plurality of magnetic field antennae;
generating a magnetic field with the plurality of magnetic field antennae;
detecting the magnetic fields generated by the plurality of magnetic field antennae at a plurality of positions within the generated magnetic field;
calculate a difference between an expected magnetic field and the detected magnetic field at each of the plurality of positions to identify any interference at the plurality of positions;
interpolating the calculated difference between each of the plurality of positions to generate a UHD interference map; and
adding the UHD interference map to the simulated UHD map.

2. The method of claim 1, further comprising placing the plurality of magnetic field antennae on a surgical bed.

3. The method of claim 2, wherein the surgical bed incudes metal components; preferably wherein the generation of magnetic fields in proximity of the metal components induces eddy currents in the metal components which contribute to the detected interference.

4. The method of any preceding claim, wherein adding the UHD interference map to the simulated UHD map generates an in-situ UHD map; preferably wherein the in-situ UHD map has a resolution of about 1 mm.

5. The method of claim 4, wherein the in-situ UHD map is stored in a memory accessible by one or more components of an electromagnetic navigation system; preferably wherein the in-situ UHD map is used to determine the position of a sensor placed within the magnetic field generated by the plurality of magnetic field antennae.

6. A method of determining a position of an electromagnetic (EM) sensor comprising:
generating a magnetic field with a plurality of magnetic field antennae;
storing in memory an ultra-high definition (UHD) map of the magnetic field generated by the plurality of magnetic field antennae;
placing an EM sensor within the magnetic field;
sampling the magnetic field with the EM sensor at one point;
conducting a global search of the UHD map of the magnetic field to identify a point in the magnetic field with the smallest difference from the point sampled by the EM sensor;
conducting a local search of a portion of the UHD map in proximity to identified point to determine a new point in the magnetic field with the smallest difference from the point sampled by the EM sensor;
iterating the local search until the difference between the new point in the magnetic field and the sampled point is below a threshold.

7. The method of claim 6, wherein upon determining that there is no convergence during the iteration of the local search, a new global search is conducted.

8. The method of claim 6 or claim 7, wherein at predetermined times a global search is performed.

9. The method of any of claims 6 to 8, wherein upon determination that difference between the new point in the magnetic field and the sampled point is below a threshold, the new point is stored as the location and orientation of the EM sensor within the magnetic field; preferably wherein the stored location and orientation of the EM sensor is used as the starting point for a further local search to identify the next location and orientation of the EM sensor.

10. The method of claim 9, wherein the location and orientation of the EM sensor is output to a user-interface for display to a user.

11. The method of claim 10, wherein the location and orientation of the EM sensor is displayed in a 3D model that has been registered to the magnetic field.

12. The method of claim 11, wherein the 3D model is of at least a portion of a patient's anatomy.

13. The method of claim 12, wherein the EM sensor is located within the portion of the patient's anatomy from which the 3D model was made.

14. The method of any of claims 6 to 13, wherein the UHD map is an in-situ UHD map that accounts for interference caused by placement of the plurality of magnetic field antennae in proximity of a metal object.

15. The method of ay of claims 6 to 14, wherein the global search is performed using a subset of the UHD map which approximates a lower resolution map.
